# EUROPEAN PATENT APPLICATION

(11) **EP 4 663 726 A1**
(43) Date of publication of application: **17.12.2025**
(21) Application number: 24753275.7
(22) Date of filing: 02.02.2024
(51) Int. Cl.: C11B 9/00, A61K 8/37, A61Q 13/00, C07C 67/36, C07C 69/75

(54) **FRAGRANCE COMPOSITION, AND METHOD FOR PRODUCING 1-ETHYLCYCLOHEXANECARBOXYLIC ACID ESTER**

(30) Priority: 07.02.2023 JP 2023016831
(71) Applicant: MITSUBISHI GAS CHEMICAL COMPANY, INC., Chiyoda-ku Tokyo 100-8324 (JP)
(72) Inventor: SHISHIMI Toru, Tokyo 100-8324 (JP); OHNO Satoshi, Tokyo 131-8501 (JP); KASHIWAGI Yuki, Wakayama-shi, Wakayama 640-8580 (JP)
(74) Representative: Müller-Boré & Partner Patentanwälte PartG mbB
(86) International application number: PCT/JP2024/003571
(87) International publication number: WO 2024/166832

(57) **Abstract**

Provided are a fragrance composition containing a 1-ethylcyclohexanecarboxylic acid ester represented by General Formula (1) as an active component, use of a 1-ethylcyclohexanecarboxylic acid ester as a fragrance, and a method for producing a 1-ethylcyclohexanecarboxylic acid ester. The fragrance composition has an impression of an herbal scent with excellent diffusibility of the scent because it contains as an active component a 1-ethylcyclohexanecarboxylic acid ester that can impart an herbal aroma and provide further diffusibility of the scent. In Formula (1), R¹ represents an alkyl group having from 1 to 3 carbons.

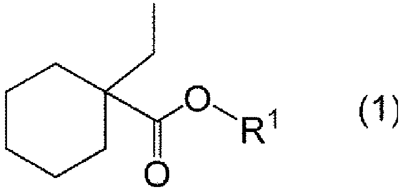

## Description

### Technical Field

The present invention relates to a fragrance composition and a method for producing a 1-ethylcyclohexanecarboxylic acid ester.

### Background Art

Ester compounds have been widely used as fragrances. In particular, formate, acetate, and the like widely exist in nature, and many of these have fruit-like scents. Synthetic fragrances that are ester compounds have been also known.

For example, Non-Patent Document 1 describes that methyl 1-methyl-3-cyclohexenecarboxylate has a chrysanthemum-like or thujone-like complex aroma and is used as a fragrance.

### Citation List

### Non-Patent Literature

Non-Patent Literature 1: Motoichi Indo, "Zoho Kaitei ban, Gosei Koryo: Kagaku to Shohin Chishiki (Enlarged and Revised Edition, Synthetic Fragrance: Chemistry and Product Knowledge)", The Chemical Daily Co. Ltd., 1996, page 672

### Summary of Invention

### Technical Problem

As described above, although many ester compounds have been used as natural fragrances or synthetic fragrances, a novel scent is required to increase value of products, such as fragrance products, cosmetic products, detergents, sanitary products, miscellaneous goods, pharmaceuticals, and food products. Typically, a fragrance is used as a compounded fragrance obtained by mixing a plurality of fragrances, and a fragrance component exhibiting an excellent effect especially when mixed with another fragrance is demanded.

Furthermore, a scent is affected also by impurities and the like generated in production process, and thus a production method for providing a fragrance having an excellent scent while being industrially practical is demanded.

An object of the present invention is to provide a fragrance composition having novel scent and characteristics, use of a compound that can impart a novel scent and characteristics to the fragrance composition, a perfuming method, and a method for producing the compound.

### Solution to Problem

The inventors of the present invention found a fragrance composition that achieves a novel scent and characteristics by containing a specific ester compound as an active component, the ester compound being excellent as a fragrance, and an efficient production method of the ester compound, and thus completed the present invention.

That is, the present invention is as follows.
[1] A fragrance composition, including a 1-ethylcyclohexanecarboxylic acid ester represented by General Formula (1) as an active component: 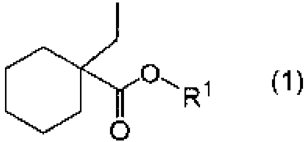 where in Formula (1), R¹ represents an alkyl group having from 1 to 3 carbons.
[2] The fragrance composition according to [1], further containing a fragrance substance other than the 1-ethylcyclohexanecarboxylic acid ester represented by Formula (1).
[3] The fragrance composition according to [2], wherein the fragrance substance other than the 1-ethylcyclohexanecarboxylic acid ester represented by Formula (1) is at least one selected from the group consisting of hydrocarbons, alcohols, phenols, aldehydes, ketones, acetals, ketals, ethers, nitriles, lactones, natural essential oils, natural extracts, and esters other than the 1-ethylcyclohexanecarboxylic acid ester represented by Formula (1).
[4] The fragrance composition according to any one of [1] to [3], wherein R¹ is at least one selected from the group consisting of a methyl group, an ethyl group, an n-propyl group, and an isopropyl group.
[5] The fragrance composition according to any one of [1] to [4], wherein R¹ is an ethyl group.
[6] Use of a 1-ethylcyclohexanecarboxylic acid ester represented by General Formula (1) as a fragrance: 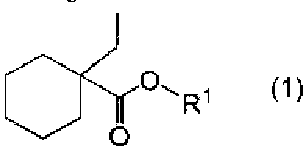 where in Formula (1), R¹ represents an alkyl group having from 1 to 3 carbons.
[7] The use according to [6], wherein R¹ is at least one selected from the group consisting of a methyl group, an ethyl group, an n-propyl group, and an isopropyl group.
[8] The use according to [6] or [7], wherein the 1-ethylcyclohexanecarboxylic acid ester represented by Formula (1) imparts an herbal scent.
[9] The use according to any one of [6] to [8], wherein R¹ is an ethyl group.
[10] The use according to [9], wherein the 1-ethylcyclohexanecarboxylic acid ester represented by Formula (1) imparts an herbal scent and a fruity scent.
[11] A perfuming method for imparting an herbal scent by a 1-ethylcyclohexanecarboxylic acid ester represented by Formula (1a): 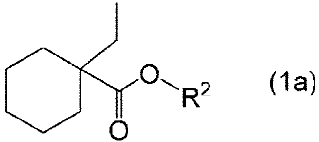 where in Formula (1a), R² represents at least one selected from the group consisting of a methyl group, an ethyl group, an n-propyl group, and an isopropyl group.
[12] The perfuming method according to [11], wherein R² is an ethyl group, and the 1-ethylcyclohexanecarboxylic acid ester represented by Formula (1a) described above imparts an herbal scent and a fruity scent.
[13] A method for producing a 1-ethylcyclohexanecarboxylic acid ester, the method comprising reacting a compound represented by General Formula (2) with carbon monoxide and an alcohol represented by General Formula (3) in the presence of hydrogen fluoride to obtain a 1-ethylcyclohexanecarboxylic acid ester represented by General Formula (1): 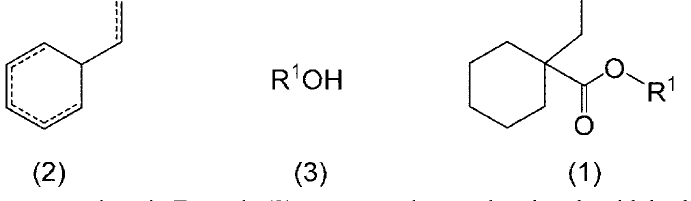 where in Formula (2), among carbon-carbon bonds with broken lines, one is a double bond, and the other bonds are single bonds; in Formula (3), R¹ represents an alkyl group having from 1 to 3 carbons; and in Formula (1), R¹ is the same as R¹ in Formula (3) and represents an alkyl group having from 1 to 3 carbons.
[14] The method for producing a 1-ethylcyclohexanecarboxylic acid ester according to [13], wherein R¹ is at least one selected from the group consisting of a methyl group, an ethyl group, an n-propyl group, and an isopropyl group.
[15] The method for producing a 1-ethylcyclohexanecarboxylic acid ester according to [13] or [14], wherein R¹ is an ethyl group.
[16] The method for producing a 1-ethylcyclohexanecarboxylic acid ester according to any one of [13] to [15], wherein the compound represented by General Formula (2) described above is at least one selected from the group consisting of vinylcyclohexane and 4-ethylcyclohexene.
[17] The method for producing a 1-ethylcyclohexanecarboxylic acid ester according to any one of [13] to [16], wherein the method includes obtaining 4-ethylcyclohexene by subjecting 4-vinylcyclohexene to hydrogenation, and the compound represented by General Formula (2) is 4-ethylcyclohexene.

### Advantageous Effects of Invention

According to the present invention, it is possible to provide: a fragrance composition having an impression of an herbal scent with excellent diffusibility of the scent because it contains as an active component a 1-ethylcyclohexanecarboxylic acid ester that can impart an herbal aroma and provide further diffusibility of the scent, use of the 1-ethylcyclohexanecarboxylic acid ester as a fragrance, and a method for producing the 1-ethylcyclohexanecarboxylic acid ester.

### Description of Embodiments

Hereinafter, in the present description, the expression "from XX to YY" means "XX or more and YY or less", "XX or higher and YY or lower", or "XX or greater and YY or less".

### [Fragrance Composition]

The fragrance composition of the present invention is a fragrance composition containing a 1-ethylcyclohexanecarboxylic acid ester represented by the following General Formula (1) as an active component.

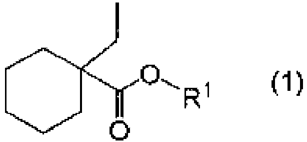

In Formula (1), R¹ represents an alkyl group having from 1 to 3 carbons.

The fragrance composition of the present invention has an impression of an herbal feel with excellent diffusibility of the scent because it contains a 1-ethylcyclohexanecarboxylic acid ester that can impart an herbal scent and provide further diffusibility of the scent as an active component. Thus, the fragrance composition is useful as an aromatic component of various products.

### <1-Ethylcyclohexanecarboxylic Acid Ester>

In Formula (1), R¹ represents an alkyl group having from 1 to 3 carbons. Specifically, R¹ is preferably at least one selected from the group consisting of a methyl group, an ethyl group, an n-propyl group, and an isopropyl group, more preferably at least one selected from the group consisting of a methyl group and an ethyl group, and even more preferably an ethyl group.

A case where R¹ is an ethyl group is remarkably useful as an active component of a fragrance composition because a fruity scent is achieved in addition to an herbal scent.

The 1-ethylcyclohexanecarboxylic acid ester represented by the aforementioned Formula (1) is useful as an active component of a fragrance composition, has an herbal scent, can impart an herbal scent to a fragrance composition, and can impart diffusibility of the scent.

When R¹ is a methyl group, an herbal scent with especially good diffusibility is achieved.

When R¹ is an ethyl group, an herbal scent with especially good diffusibility and a fruity scent are achieved.

When R¹ is an n-propyl group, an herbal scent with especially good diffusibility is achieved.

When R¹ is an isopropyl group, an herbal scent with especially good diffusibility is achieved.

The content of the 1-ethylcyclohexanecarboxylic acid ester in the fragrance composition of the present invention is may be appropriately adjusted according to the type of fragrance composition, the type of target aroma, the intensity of the aroma, and the like and is preferably from 0.01 to 90 mass%, more preferably from 0.1 to 50 mass%, even more preferably from 0.02 to 30 mass%, yet even more preferably from 0.05 to 20 mass%, yet even more preferably from 0.1 to 10 mass%, yet even more preferably from 0.5 to 10 mass%, and yet even more preferably from 0.8 to 7 mass%.

### <Additional Component and Product That Can Be Used>

Examples of an additional component that is contained in the fragrance composition containing the 1-ethylcyclohexanecarboxylic acid ester and that is other than the 1-ethylcyclohexanecarboxylic acid ester include fragrance substances other than the 1-ethylcyclohexanecarboxylic acid ester represented by Formula (1), surfactants, solvents, antioxidants, and coloring agents. The additional component is preferably at least one selected from the group consisting of fragrance substances other than the 1-ethylcyclohexanecarboxylic acid ester represented by Formula (1), surfactants, solvents, antioxidants, and coloring agents, more preferably at least one selected from the group consisting of fragrance substances other than the 1-ethylcyclohexanecarboxylic acid ester represented by Formula (1) and solvents, and even more preferably fragrance substances other than the 1-ethylcyclohexanecarboxylic acid ester represented by Formula (1). That is, a preferred fragrance composition of the present invention contains the 1-ethylcyclohexanecarboxylic acid ester represented by Formula (1) as an active component and contains a fragrance substance other than the 1-ethylcyclohexanecarboxylic acid ester represented by Formula (1).

The inclusion of a fragrance substance other than the 1-ethylcyclohexanecarboxylic acid ester represented by Formula (1) allows the scent to be adjusted to accommodate a target product. In addition, the inclusion of a solvent allows for easy dissolution into and impregnation of a target product, making it possible to adjust the intensity of the scent or the durability of the scent.

In particular, a fragrance substance other than the 1-ethylcyclohexanecarboxylic acid ester represented by Formula (1) preferably constitutes a floral-type fragrance composition, and the 1-ethylcyclohexanecarboxylic acid ester represented by Formula (1) is preferably contained as an active component of the floral-type fragrance composition.

The fragrance substance other than the 1-ethylcyclohexanecarboxylic acid ester represented by Formula (1) is not particularly limited as long as it is a known fragrance component, and a wide range of fragrances can be used. For example, one, or two or more of the following fragrance substances can be selected and used at any mixing ratio.

The fragrance substance other than the 1-ethylcyclohexanecarboxylic acid ester represented by Formula (1) is preferably at least one selected from the group consisting of hydrocarbons, alcohols, phenols, aldehydes, ketones, acetals, ketals, ethers, nitriles, lactones, natural essential oils, natural extracts, and esters other than the 1-ethylcyclohexanecarboxylic acid ester represented by Formula (1), more preferably at least one selected from the group consisting of alcohols, aldehydes, and esters other than the 1-ethylcyclohexanecarboxylic acid ester represented by Formula (1), and even more preferably at least one selected from the group consisting of alcohols and aldehydes.

Examples of hydrocarbons include limonene, α-pinene, β-pinene, terpinene, cedrene, longifolene, and valencene.

Examples of alcohols include linalool, citronellol, geraniol, nerol, terpineol, dihydromyrcenol, ethyllinalool, farnesol, nerolidol, cis-3-hexenol, cedrol, menthol, borneol, β-phenylethyl alcohol, benzyl alcohol, phenyl hexanol, 2,2,6-trimethylcyclohexyl-3-hexanol, 1-(2-t-butylcyclohexyloxy)-2-butanol, 4-isopropylcyclohexane methanol, 4-t-butylcyclohexanol, 4-methyl-2-(2-methylpropyl)tetrahydro-2H-pyran-4-ol, 2-methyl-4-(2,2,3-trimethyl-3-cyclopenten-1-yl)-2-buten-1-ol, 2-ethyl-4-(2,2,3-trimethyl-3-cyclopenten-1-yl)-2-buten-1-ol, isocamphylcyclohexanol, and 3,7-dimethyl-7-methoxyoctan-2-ol.

Examples of phenols include eugenol, thymol, and vanillin.

Examples of esters include linalyl formate, citronellyl formate, geranyl formate, n-hexyl acetate, cis-3-hexenyl acetate, linalyl acetate, citronellyl acetate, geranyl acetate, nellyl acetate, terpinyl acetate, nopil acetate, bornyl acetate, isobornyl acetate, o-t-butylcyclohexyl acetate, p-t-butylcyclohexyl acetate, tricyclodecenyl acetate, benzyl acetate, styralyl acetate, cinnamyl acetate, dimethylbenzyl carbinyl acetate, 3-pentyltetrahydropyran-4-yl acetate, citronellyl propionate, tricyclodecenyl propionate, allylcyclohexyl propionate, ethyl-2-cyclohexyl propionate, benzyl propionate, citronellyl butyrate, dimethylbenzylcarbinyl n-butyrate, tricyclodecenyl isobutyrate, methyl-2-nonenoate, methyl benzoate, benzyl benzoate, methyl cinnamate, methyl salicylate, n-hexyl salicylate, cis-3-hexenyl salicylate, geranyl tiglate, cis-3-hexenyl tiglate, methyl jasmonate, methyldihydro jasmonate, methyl-2,4-dihydroxy-3,6-dimethyl benzoate, ethylmethylphenyl glycidate, methyl anthranilate, and fruitate.

Examples of aldehydes include n-octanal, n-decanal, n-dodecanal, 2-methylundecanal, 10-undecenal, citronellal, citral, hydroxycitronellal, dimethyl tetrahydrobenzaldehyde, 4-(4-hydroxy-4-methylpentyl)-3-cyclohexene-1-carboaldehyde, 2-cyclohexyl propanal, p-t-butyl-α-methylhydrocinnamic aldehyde, p-t-butylhydrocinnamic aldehyde, p-isopropyl-α-methylhydrocinnamic aldehyde, p-ethyl-α,α-dimethylhydrocinnamic aldehyde, α-amylcinnamic aldehyde, α-hexylcinnamic aldehyde, piperonal, and α-methyl-3,4-methylenedioxyhydrocinnamic aldehyde.

Examples of ketones include methylheptenone, 4-methylene-3,5,6,6-tetramethyl-2-heptanone, amylcyclopentanone, 3-methyl-2-(cis-2-penten-1-yl)-2-cyclopenten-1-one, methylcyclopentenolone, rose ketone, γ-methylionone, α-ionone, carbone, menthone, camphor, nootkatone, benzylacetone, anysilacetone, methyl-β-naphthylketone, 2,5-dimethyl-4-hydroxy-3(2H)-furanone, maltol, 7-acetyl-1,2,3,4,5,6,7,8-octahydro-1,1,6,7-tetramethyl naphthalene, muscone, civetone, cyclopentadecanone, and cyclohexedecenone.

Examples of acetals and ketals include acetoaldehyde ethylphenylpropyl acetal, citraldiethyl acetal, phenylacetoaldehyde glycerin acetal, and ethylacetoacetate ethylene glycol ketal.

Examples of ethers include anetol, β-naphthylmethyl ether, β-naphthylethyl ether, limonene oxide, rose oxide, 1,8-cineol, racemic or optically active dodecahydro-3a,6,6,9a-tetramethylnaphtho[2,1-b]furane.

Examples of nitriles include citronellyl nitrile.

Examples of lactones include γ-nonalactone, γ-undecalactone, σ-decalactone, γ-jasmolactone, coumarin, cyclopentadecanolide, cyclohexadecanolide, ambrettolide, ethylene brassylate, and 11-oxahexadecanolide.

Examples of natural essential oils and natural extracts include those of orange, lemon, bergamot, mandarin, peppermint, spearmint, lavender, chamomile, rosemary, eucalyptus, sage, basil, rose, geranium, jasmine, ylang-ylang, anise, clove, ginger, nutmeg, cardamom, cedar, Japanese cypress, sandalwood, vetiver, patchouli, and labdanum.

Examples of the solvent include dipropylene glycol, diethyl phthalate, ethylene glycol, propylene glycol, methyl myristate, and triethyl citrate.

Examples of the surfactant include polyoxyethylene lauryl sulfate ether.

The fragrance composition containing the 1-ethylcyclohexanecarboxylic acid ester represented by General Formula (1) can be used as an aromatic component of various products.

The products in which the fragrance composition can be used include: fragrance products, such as perfumes and colognes; cosmetics for hair, such as shampoos, conditioners, hair tonics, hair creams, mousses, gels, pomades, and sprays; cosmetics for skin, such as face lotions, serums, creams, emulsions, facial masks, foundations, face powders, lipsticks, and various types of makeup; various detergents for health and sanitation, such as dish detergents, laundry detergents, softeners, disinfectant detergents, odor eliminating detergents, indoor aromatic agent, furniture care products, glass cleaners, furniture cleaners, floor cleaners, disinfectants, insecticides, bleaches, germicides, and repellents; quasi-drugs, such as toothpaste, mouthwash, bath salts, antiperspirants, and perm solutions; miscellaneous goods such as toilet paper and tissue paper; pharmaceuticals; and food products. Among these, the fragrance composition containing the 1-ethylcyclohexanecarboxylic acid ester represented by General Formula (1) is preferably used for a toiletry product such as detergents for clothing and detergents for bodies.

The amount of the fragrance composition blended in the product described above is not particularly limited and can be selected depending on the type, nature, and sensory effect of the product to be perfumed. For example, the amount of the fragrance composition blended in the product is preferably 0.00001 mass% or greater, more preferably 0.0001 mass% or greater, and even more preferably 0.001 mass% or greater. Also, the amount of the fragrance composition blended in the product is preferably 80 mass% or less, more preferably 60 mass% or less, and even more preferably 40 mass% or less.

Note that, when the fragrance composition is used as a fragrance oil, perfume, or the like, the amount of the fragrance composition blended in the product may be 80 mass% or greater, or may be 100 mass%.

### [Use as Fragrance]

Because the 1-ethylcyclohexanecarboxylic acid ester represented by the following General Formula (1) has an excellent herbal scent, the 1-ethylcyclohexanecarboxylic acid ester can be used as a fragrance. Use of the 1-ethylcyclohexanecarboxylic acid ester represented by the following General Formula (1) as a fragrance is also included in the present invention.

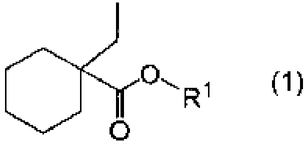

In Formula (1), R¹ represents an alkyl group having from 1 to 3 carbons.

In Formula (1), R¹ represents an alkyl group having from 1 to 3 carbons. Specifically, R¹ is preferably at least one selected from the group consisting of a methyl group, an ethyl group, an n-propyl group, and an isopropyl group, more preferably at least one selected from the group consisting of a methyl group and an ethyl group, and even more preferably an ethyl group.

When R¹ is an ethyl group, use as a fragrance is more suitable because a fruity scent is achieved in addition to an herbal scent.

The 1-ethylcyclohexanecarboxylic acid ester represented by the aforementioned Formula (1) has an excellent herbal scent. Thus, use of the 1-ethylcyclohexanecarboxylic acid ester represented by Formula (1) imparts an herbal scent. Because a fruity scent is achieved in addition to an herbal scent when R¹ is an ethyl group, in a case where R¹ is an ethyl group, use of the 1-ethylcyclohexanecarboxylic acid ester represented by Formula (1) imparts the herbal scent and the fruity scent.

When R¹ is a methyl group, an herbal scent with especially good diffusibility is achieved.

When R¹ is an ethyl group, an herbal scent with especially good diffusibility and a fruity scent are achieved.

When R¹ is an n-propyl group, an herbal scent with especially good diffusibility is achieved.

When R¹ is an isopropyl group, an herbal scent with especially good diffusibility is achieved.

The 1-ethylcyclohexanecarboxylic acid ester represented by Formula (1) can be used as an aromatic component of various products. Note that, in a case where the 1-ethylcyclohexanecarboxylic acid ester represented by Formula (1) is used as aromatic component for various products, the 1-ethylcyclohexanecarboxylic acid ester may be used as one component of the fragrance composition.

The products in which the 1-ethylcyclohexanecarboxylic acid ester represented by Formula (1) can be used include: fragrance products, such as perfumes and colognes; cosmetics for hair, such as shampoos, conditioners, hair tonics, hair creams, mousses, gels, pomades, and sprays; cosmetics for skin, such as face lotions, serums, creams, emulsions, facial masks, foundations, face powders, lipsticks, and various types of makeup; various detergents for health and sanitation, such as dish detergents, laundry detergents, softeners, disinfectant detergents, odor eliminating detergents, indoor aromatic agent, furniture care products, glass cleaners, furniture cleaners, floor cleaners, disinfectants, insecticides, bleaches, germicides, and repellents; quasi-drugs, such as toothpaste, mouthwash, bath salts, antiperspirants, and perm solutions; miscellaneous goods such as toilet paper and tissue paper; pharmaceuticals; and food products.

The amount of the 1-ethylcyclohexanecarboxylic acid ester represented by Formula (1) blended in the product described above is not particularly limited and can be selected depending on the type, nature, and sensory effect of the product to be perfumed. For example, the amount of the 1-ethylcyclohexanecarboxylic acid ester represented by Formula (1) blended in the product is preferably 0.00001 mass% or greater, more preferably 0.0001 mass% or greater, and even more preferably 0.001 mass% or greater. Also, the amount of the 1-ethylcyclohexanecarboxylic acid ester represented by Formula (1) blended in the product is preferably 80 mass% or less, more preferably 60 mass% or less, and even more preferably 40 mass% or less.

Note that, when the 1-ethylcyclohexanecarboxylic acid ester represented by Formula (1) is used as a fragrance oil, perfume, or the like, the amount of the 1-ethylcyclohexanecarboxylic acid ester represented by Formula (1) blended in the product may be 80 mass% or greater, or may be 100 mass%.

### [Perfuming Method]

Because the 1-ethylcyclohexanecarboxylic acid ester represented by General Formula (1a) described below has an excellent herbal scent, the 1-ethylcyclohexanecarboxylic acid ester can impart an herbal scent to various products. A perfuming method for imparting an herbal scent by the 1-ethylcyclohexanecarboxylic acid ester represented by General Formula (1a) described below is also included in the present invention.

A method of imparting an herbal scent to a product may be coating, spraying, dipping, or the like in a case where the product is a solid and may be mixing in a case where the product is a liquid. Note that, in a case where an herbal scent is imparted by the 1-ethylcyclohexanecarboxylic acid ester represented by Formula (1a), the herbal scent may be imparted to various products by using the 1-ethylcyclohexanecarboxylic acid ester represented by Formula (1a) as a component in the fragrance composition.

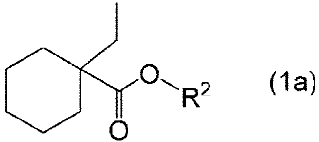

In Formula (1a), R² represents at least one selected from the group consisting of a methyl group, an ethyl group, an n-propyl group, and an isopropyl group.

In Formula (1a), R¹ is at least one selected from the group consisting of a methyl group, an ethyl group, an n-propyl group, and an isopropyl group, more preferably at least one selected from the group consisting of a methyl group and an ethyl group, and even more preferably an ethyl group.

When R¹ is an ethyl group, an herbal scent and a fruity scent can be imparted to various products because the fruity scent is achieved in addition to the herbal scent.

Because the 1-ethylcyclohexanecarboxylic acid ester represented by the aforementioned Formula (1a) has an excellent herbal scent, the 1-ethylcyclohexanecarboxylic acid ester can impart an herbal scent to various products. Because a fruity scent is achieved in addition to an herbal scent when R¹ is an ethyl group, in a case where R¹ is an ethyl group, the 1-ethylcyclohexanecarboxylic acid ester represented by Formula (1a) can impart an herbal scent and a fruity scent to various products.

When R¹ is a methyl group, an herbal scent with especially good diffusibility is achieved, and the herbal scent is imparted to products.

When R¹ is an ethyl group, an herbal scent with especially good diffusibility and a fruity scent are achieved, and the herbal scent and the fruity scent are imparted to products.

When R¹ is an n-propyl group, an herbal scent with especially good diffusibility is achieved, and the herbal scent is imparted to products.

When R¹ is an isopropyl group, an herbal scent with especially good diffusibility is achieved, and the herbal scent can be imparted to products.

The products to which an herbal scent can be imparted by the 1-ethylcyclohexanecarboxylic acid ester represented by Formula (1a) include: fragrance products, such as perfumes and colognes; cosmetics for hair, such as shampoos, conditioners, hair tonics, hair creams, mousses, gels, pomades, and sprays; cosmetics for skin, such as face lotions, serums, creams, emulsions, facial masks, foundations, face powders, lipsticks, and various types of makeup; various detergents for health and sanitation, such as dish detergents, laundry detergents, softeners, disinfectant detergents, odor eliminating detergents, indoor aromatic agent, furniture care products, glass cleaners, furniture cleaners, floor cleaners, disinfectants, insecticides, bleaches, germicides, and repellents; quasi-drugs, such as toothpaste, mouthwash, bath salts, antiperspirants, and perm solutions; miscellaneous goods such as toilet paper and tissue paper; pharmaceuticals; and food products.

The amount of the 1-ethylcyclohexanecarboxylic acid ester represented by Formula (1a) blended in the product described above is not particularly limited and can be selected depending on the type, nature, and sensory effect of the product to be perfumed. For example, the amount of the 1-ethylcyclohexanecarboxylic acid ester represented by Formula (1a) blended in the product is preferably 0.00001 mass% or greater, more preferably 0.0001 mass% or greater, and even more preferably 0.001 mass% or greater. Also, the amount of the 1-ethylcyclohexanecarboxylic acid ester represented by Formula (1a) blended in the product is preferably 80 mass% or less, more preferably 60 mass% or less, and even more preferably 40 mass% or less.

Note that, when the 1-ethylcyclohexanecarboxylic acid ester represented by Formula (1a) is used as a fragrance oil, perfume, or the like, the amount of the 1-ethylcyclohexanecarboxylic acid ester represented by Formula (1a) blended in the product may be 80 mass% or greater, or may be 100 mass%.

### [Method for Producing 1-Ethylcyclohexanecarboxylic Acid Ester]

The method for producing the 1-ethylcyclohexanecarboxylic acid ester contained in the fragrance composition of the present invention as the active component is not limited but is preferably the following production method. Note that the 1-ethylcyclohexanecarboxylic acid ester obtained in the following production method is preferably used as a fragrance as described above and is also preferably used in the perfuming method. Furthermore, the following production method is also included in the present invention.

The method for producing a 1-ethylcyclohexanecarboxylic acid ester according to the present invention is a method for producing a 1-ethylcyclohexanecarboxylic acid ester, the method including reacting a compound represented by General Formula (2) with carbon monoxide and an alcohol represented by General Formula (3) in the presence of hydrogen fluoride to obtain a 1-ethylcyclohexanecarboxylic acid ester represented by General Formula (1).

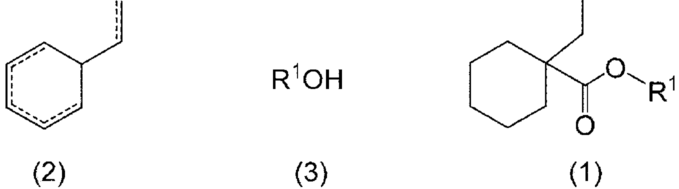

In Formula (2), among carbon-carbon bonds with broken lines, one is a double bond, and the other bonds are single bonds. In Formula (3), R¹ represents an alkyl group having from 1 to 3 carbons. In Formula (1), R¹ is the same as R¹ in Formula (3) and represents an alkyl group having from 1 to 3 carbons.

### <Compound Represented by Formula (2)>

The compound represented by Formula (2) is any one of a compound represented by the following Formula (2a), a compound represented by the following Formula (2b), or a compound represented by the following Formula (2c); preferably at least one selected from the group consisting of a compound represented by the following Formula (2a) and a compound represented by the following Formula (2b); and more preferably a compound represented by the following Formula (2a).

The compound represented by Formula (2a) is 4-ethylcyclohexene, and the compound represented by Formula (2b) is vinylcyclohexane. Thus, the compound represented by Formula (2) is preferably at least one selected from the group consisting of vinylcyclohexane and 4-ethylcyclohexene, and is more preferably 4-ethylcyclohexene.

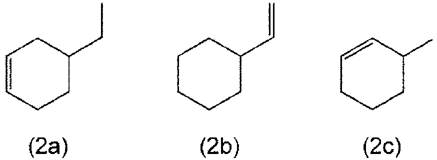

The compound represented by Formula (2a) may be obtained by subjecting 4-vinylcyclohexene to hydrogenation. That is, the present production method preferably includes, in a case in which the compound represented by Formula (2) is 4-ethylcyclohexene, obtaining 4-ethylcyclohexene by subjecting 4-vinylcyclohexene to hydrogenation. The reaction scheme is presented below.

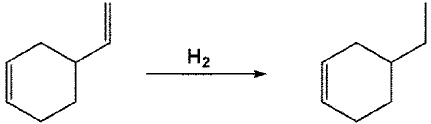

The reaction conditions of the hydrogenation are not particularly limited and maybe reaction conditions that are typically used for hydrogenation of an unsaturated bond.

A catalyst is preferably used for the hydrogenation. The catalyst is not particularly limited as long as it is a hydrogenation catalyst and is preferably a catalyst containing at least one type selected from Group 8 to 11 metals.

Specific examples thereof include a catalyst containing at least one type of iron, cobalt, nickel, copper, ruthenium, rhodium, palladium, silver, osmium, iridium, platinum, or gold.

The hydrogenation catalyst may be a solid catalyst or a homogeneous catalyst, and is preferably a solid catalyst from the viewpoint of separability from a reaction product.

Examples of the solid catalyst include unsupported metal catalysts and supported metal catalysts. The unsupported metal catalyst is preferably Raney catalysts, such as Raney nickel, Raney cobalt, and Raney copper, oxides of platinum, palladium, rhodium, and ruthenium, and colloid catalysts.

Examples of the supported metal catalyst include catalysts obtained by allowing at least one type of iron, cobalt, nickel, copper, ruthenium, rhodium, palladium, silver, osmium, iridium, platinum, or gold to be supported on or mixed in carriers such as magnesia, zirconia, ceria, diatomaceous earth, active carbon, alumina, silica, zeolite, or titania. A supported copper catalyst, in which a copper catalyst, such as a copper-chromium catalyst (Adkins catalyst), a copper-zinc catalyst, or copper-iron, is supported on a carrier; a supported platinum catalyst such as Pt/C or Pt/alumina; a supported palladium catalyst such as Pd/C or Pd/alumina; a supported ruthenium catalyst such as Ru/C or Ru/alumina; and a supported rhodium catalyst such as Rh/C or Rh/alumina are preferred. Among these, from the viewpoints of reaction activity and selectivity, use of a catalyst containing copper is more preferred.

The amount of the hydrogenation catalyst to be used varies depending on the type of the catalyst but is suitably from 0.001 to 100 mass%, preferably from 0.01 to 30 mass%, and even more preferably from 0.1 to 20 mass%, with respect to the amount of the 4-vinylcyclohexene, which is a raw material.

The pressure of the hydrogen may be at normal pressure or increased pressure and is typically in a range of 0.1 to 4.0 MPa, preferably in a range of 0.1 to 3.0 MPa, and even more preferably in a range of 0.1 to 2.0 MPa.

Although the hydrogenation reaction can be performed solvent free, a solvent may be used. Examples of the solvent include esters such as ethyl acetate and butyl acetate; aromatic compounds such as benzene, o-dichlorobenzene, toluene, and xylene; hydrocarbons such as hexane, heptane, and cyclohexane; alcohols such as methanol, ethanol, isopropyl alcohol, t-butyl alcohol, ethylene glycol, and diethylene glycol; ethers such as dioxane, tetrahydrofuran, dimethoxyethane, and diglyme, and mixtures of these.

The amount of the solvent to be used in the hydrogenation reaction is typically from 0.1 to 30 mass times, and preferably from 0.2 to 20 mass times, of the amount of the 4-vinylcyclohexene, which is a raw material.

The reaction temperature of the hydrogenation reaction is typically from -90°C to 200°C, preferably from 20°C to 150°C, and more preferably from 20°C to 100°C.

The form of the hydrogenation reaction is not particularly limited as long as catalytic hydrogenation reaction can be performed and may be a known form that is typically used. Examples thereof include a slurry-bed reactor for performing catalytic hydrogenation reaction by allowing the catalyst to be fluidized by a fluid, and a fixed-bed reactor for performing catalytic hydrogenation reaction by supplying a fluid while the catalyst is charged and fixed.

### <Alcohol Represented by Formula (3)>

The alcohol represented by Formula (3) is a monohydric alcohol including an alkyl group having from 1 to 3 carbons. R¹ of Formula (3) is an alkyl group having from 1 to 3 carbons. Specifically, R¹ is preferably at least one selected from the group consisting of a methyl group, an ethyl group, an n-propyl group, and an isopropyl group, more preferably at least one selected from the group consisting of a methyl group and an ethyl group, and even more preferably an ethyl group. Specifically, the alcohol represented by Formula (3) is preferably methanol, ethanol, 1-propanol, or 2-propanol, and is more preferably ethanol.

### <Carbonylation Reaction>

The production method of the present invention is a method by which the 1-ethylcyclohexanecarboxylic acid ester represented by Formula (1) is obtained by reacting a compound represented by Formula (2) with carbon monoxide and an alcohol represented by Formula (3) in the presence of hydrogen fluoride. First, carbonylation occurs using carbon monoxide, and then the obtained carbonyl compound and an alcohol are reacted to obtain the 1-ethylcyclohexanecarboxylic acid ester represented by Formula (1).

First, the carbonylation reaction will be described, and then conversion into an ester will be described.

Because the hydrogen fluoride used in the carbonylation reaction is a solvent for reaction, is a catalyst, and also is an auxiliary material, a substantially anhydrous hydrogen fluoride is used. The amount of the hydrogen fluoride to be used is preferably from 4 to 15 mole times, and more preferably from 6 to 10 mole times, the amount of the compound represented by Formula (2) of the raw material. When the molar ratio of the hydrogen fluoride is 4 mole times or greater, the reaction proceeds efficiently, side reactions such as disproportionation and polymerization can be suppressed, and the 1-ethylcyclohexanecarboxylic acid ester represented by Formula (1), which is the target product, can be obtained at a high yield. Furthermore, from the viewpoint of material costs and productivity, the molar ratio of the hydrogen fluoride is preferably 15 mole times or less.

The carbon monoxide used in the carbonylation reaction may contain an inert gas such as nitrogen or methane, and carbon monoxide partial pressure during the reaction is preferably from 0.5 to 5 MPa, and more preferably from 1 to 3 MPa. When the carbon monoxide partial pressure is greater than 0.5 MPa, the carbonylation reaction adequately proceeds without side reactions such as disproportionation and polymerization, and the 1-ethylcyclohexanecarboxylic acid ester represented by Formula (1), which is the target product, can be obtained at a high yield. Furthermore, from the viewpoint of equipment load, the carbon monoxide partial pressure is preferably 5 MPa or less.

The carbonylation reaction may be performed in any manner, for example, in a batch, semi-continuous, or continuous manner, which is not particularly limited.

The reaction temperature of the carbonylation reaction is preferably from -50°C to 30°C, more preferably from -40°C to 0°C, and even more preferably from -50 to -25°C. The reaction temperature of the carbonylation reaction of 30°C or lower results in good yield. Furthermore, from the viewpoint of reaction rate, the reaction is preferably performed at -50°C or higher.

In the carbonylation reaction, an acid fluoride is produced by hydrogen fluoride and carbon monoxide. After excess hydrogen fluoride was distilled and removed, the produced acid fluoride reaction solution may be purified by an ordinary method, such as distillation, but is preferably converted into an ester by allowing a reaction as is with an alcohol.

### <Conversion into Ester>

As described above, in a case where an ester is synthesized by allowing a reaction between the reaction solution produced in the carbonylation reaction and an alcohol, after the acid fluoride is separated once, the reaction with the alcohol may be performed again in the presence of a hydrogen fluoride catalyst; however, preferably, the reaction with an alcohol is performed without separation of the acid fluoride to obtain an ester. From the viewpoint of causticity of the reaction equipment, at this time, a method in which a predetermined amount of alcohol is added in the acid fluoride reaction solution is preferred.

The amount of the alcohol represented by Formula (3) to be used is preferably from 0.5 to 2.0 mole times, and more preferably from 0.8 to 1.5 mole times, the amount of the compound represented by Formula (2) that is the raw material of the carbonylation process. The molar ratio of the alcohol of 0.5 mole times or greater is preferred because the amount of remained unreacted fluoride is small and less corrosion of equipment occurs in the post-process. The molar ratio is preferably 2.0 mole times or less from the viewpoint of suppression of corrosion of equipment by suppressing dehydration reaction between alcohol molecules.

The reaction temperature of the acid fluoride and the alcohol is preferably 20°C or lower from the viewpoint of suppression of ester decomposition. The reaction temperature of 20°C or lower is preferred because dehydration reaction of alcohol molecules can be suppressed.

After the hydrogen fluoride is distilled off from the obtained ester, purification is performed by an ordinary method such as distillation, and thus the 1-ethylcyclohexanecarboxylic acid ester represented by Formula (1) is obtained. Because the obtained 1-ethylcyclohexanecarboxylic acid ester has an excellent herbal scent, the 1-ethylcyclohexanecarboxylic acid ester can be suitably used as a fragrance. When the 1-ethylcyclohexanecarboxylic acid ester is contained in the fragrance composition as an active component, a fragrance composition having an impression of an herbal feel and excellent diffusibility of the scent can be obtained. Furthermore, according to the present production method, because the 1-ethylcyclohexanecarboxylic acid ester can be obtained efficiently, a fragrance having an excellent scent can be obtained in an industrially practical manner.

### Examples

The present invention will be described in detail based on Examples presented below, but the present invention is not limited to these Examples.

### Analysis and Evaluation

### Purity (GC Analysis)

The purity of the 1-ethylcyclohexanecarboxylic acid ester obtained in each of Examples 1 to 4 was analyzed by gas chromatography (GC analysis). The conditions of the GC analysis are as follows.

### GC Analysis Condition

100 mg of sample (product of Example) was diluted with 2 mL of diethyl ether (available from FUJIFILM Wako Pure Chemical Corporation). "GC-6850" available from Agilent Technologies was used as the measurement instrument, and "DB-1 (internal diameter: 0.25 mm; length: 30 m; film thickness: 0.25 µm)" available from J&W was used as a column. The temperature increase condition was 10°C/min from 80°C to 300°C.

### Identification of Compound (GC-MS Analysis and NMR Spectral Analysis)

The identification of the 1-ethylcyclohexanecarboxylic acid ester obtained in Examples 1 to 4 was performed by gas chromatography-mass spectrometry analysis (GC-MS analysis) and NMR spectral analysis. The conditions of each analysis are as follows.

### GC-MS Analysis Condition

10 mg of sample (1-ethylcyclohexanecarboxylic acid ester) was diluted with 2 mL of diethyl ether (available from FUJIFILM Wako Pure Chemical Corporation). 5977B GC/MSD available from Agilent Technologies was used as the measurement instrument, and "HP-5 (internal diameter: 0.25 mm; length: 30 m; film thickness: 0.25 µm)" available from J&W was used as a column. The temperature increase condition was 10°C/min from 80°C to 300°C. The ionization method for MSD was EI.

### NMR Spectral Analysis Condition

10 mg of sample (1-ethylcyclohexanecarboxylic acid ester) was diluted with 0.8 mL of chloroform-d (deuteration ratio: 99.8%; 0.05 vol% TMS contained; available from FUJIFILM Wako Pure Chemical Corporation) and subjected to ¹H-NMR and ¹³C-NMR measurement by Agilent-NMR-VNMRS 400, available from Varian.

### Evaluation of Scent

The 1-ethylcyclohexanecarboxylic acid esters obtained in Examples and the fragrance compositions obtained in Examples and Comparative Examples were subjected to sensory evaluation using fragrance testing paper. The sensory evaluation was performed by four experts who have not less than 5 years of evaluation experience.

### Synthesis of Starting Material

### Production Example 1 (Synthesis of 4-Ethylcyclohexene)

A stainless steel autoclave having an internal volume of 5 L, equipped with a magnetically induced stirrer, three inlet nozzles at an upper part, and one discharge nozzle equipped with a filter at a bottom part, and capable of controlling the internal temperature through a jacket was charged with 2.0 g of a Cu-Cr catalyst (product name "N-203S", available from JGC Catalysts and Chemicals Ltd.) and 100 g of heptane (guaranteed grade; available from FUJIFILM Wako Pure Chemical Corporation) and activated for 1 hour at 170°C with a hydrogen pressure of 2 MPa.

After the cooling, only heptane was discharged through the discharge nozzle equipped with the filter at the bottom part, 50 g of 4-vinylcyclohexene (available from Tokyo Chemical Industry Co., Ltd.) and 6.25 g of heptane (guaranteed grade; available from FUJIFILM Wako Pure Chemical Corporation) were charged, and hydrogenation reaction was performed by stirring at 50°C and a hydrogen pressure of 1 MPa for 5 hours. The reaction solution was filtered to remove the catalyst, the obtained reaction solution was purified by using a distillation column with the number of theoretical plates of 20, and thus 45 g of 4-ethylcyclohexene was obtained.

### Production of 1-Ethylcyclohexanecarboxylic Acid Ester

### Example 1 (Production of Ethyl 1-Ethylcyclohexanecarboxylate)

### Carbonylation Process

An experiment was performed using a stainless steel autoclave having an internal volume of 500 mL equipped with a magnetically induced stirrer, three inlet nozzles at the top, and one discharge nozzle at the bottom, the internal temperature of which can be controlled by a jacket.

First, the inside of the autoclave was purged with nitrogen, after which 120 g (6 mol) of hydrogen fluoride was introduced, and the liquid temperature was adjusted to -10°C, and subsequently, the pressure was increased to 2 MPa with carbon monoxide.

While the reaction temperature and the reaction pressure were respectively maintained at -10°C and 2 MPa, a mixed solution of 67 g (0.6 mol) of the 4-ethylcyclohexene obtained by Production Example 1 and 67 g of heptane was supplied from the upper part of the autoclave for 45 minutes, and a carbonylation reaction was carried out. After the supply was ended, stirring was continued for approximately 20 minutes until absorption of carbon monoxide was no longer observed.

### Esterification Process

Subsequently, while the reaction temperature was maintained at -10°C, 41.7 g (0.9 mol) of ethanol was supplied from the upper part of the autoclave for 15 minutes, and esterification was performed for 1 hour under stirring.

The reaction solution was discharged from the bottom of the autoclave into ice water, and the oil phase and the aqueous phase were separated, after which the oil phase was washed once with 100 mL of a 2% caustic soda aqueous solution and twice with 100 mL of distilled water, and then dehydrated with 10 g of anhydrous sodium sulfate. The obtained oil phase part was purified by using a distillation column with the number of theoretical plates of 20, and thus 78 g of ethyl 1-ethylcyclohexanecarboxylate was obtained as a colorless, transparent liquid. The result of the scent evaluation of the obtained ethyl 1-ethylcyclohexanecarboxylate is shown in Table 1.

### Ethyl 1-Ethylcyclohexanecarboxylate

As a result of the GC analysis, the purity of the main product was 99.7%. It was confirmed that the result of the GC-MS analysis of the main product was 184 (10, M⁺), 156 (25), 129 (24), 111 (100), 69 (74), 55 (25), and it matched a molecular weight of the target product of 184. Furthermore, the analysis value of ¹H-NMR (CDCl₃, 400 MHz, δ ppm) was 0.81 (t, J = 7.6 Hz, 3H), 1.10-1.40 (m, 8H), 1.46-1.61 (m, 5H), 2.01-2.11 (m, 2H), 4.15 (q, J = 7.1 Hz, 2H), and the analysis value of ¹³C-NMR (CDCl₃, 400 MHz, δ ppm) was 8.5 (CH₃), 14.3 (CH₃), 23.3 (CH₂), 26.0 (CH₂), 33.3 (CH₂), 33.8 (CH₂), 47.2 (C), 59.9 (OCH₂), 176.8 (CO), and the main product was identified as ethyl 1-ethylcyclohexanecarboxylate.

### Example 2 (Production of Methyl 1-Ethylcyclohexanecarboxylate)

72 g of methyl 1-ethylcyclohexanecarboxylate as a colorless, transparent liquid was obtained in the same manner as in Example 1 except for using 27 g (0.84 mol) of methanol in place of 41.7 g (0.9 mol) of the ethanol used in the esterification of Example 1. The result of the scent evaluation of the obtained methyl 1-ethylcyclohexanecarboxylate is shown in Table 1.

### Methyl 1-Ethylcyclohexanecarboxylate

As a result of the GC analysis, the purity of the main product was 99.8%. It was confirmed that the result of the GC-MS analysis of the main product was 170 (13, M⁺), 142 (25), 115 (44), 102 (20), 69 (100), 55 (37), and it matched a molecular weight of the target product of 170. Furthermore, the analysis value of ¹H-NMR (CDCl₃, 400 MHz, δ ppm) was 0.79 (t, J = 7.4 Hz, 3H), 1.12-1.38 (m, 5H), 1.47-1.62 (m, 5H), 2.01-2.12 (m, 2H), 3.68 (s, 3H), and the analysis value of ¹³C-NMR (CDCl₃, 400 MHz, δ ppm) was 8.6 (CH₃), 23.3 (CH₂), 26.0 (CH₂), 33.3 (CH₂), 33.8 (CH₂), 47.5 (C), 51.4 (OCH₃), 177.3 (CO), and the main product was identified as methyl 1-ethylcyclohexanecarboxylate.

### Example 3 (Production of Propyl 1-Ethylcyclohexanecarboxylate)

78 g of propyl 1-ethylcyclohexanecarboxylate as a colorless, transparent liquid was obtained in the same manner as in Example 1 except for using 49 g (0.82 mol) of I-propanol in place of 41.7 g (0.9 mol) of the ethanol used in the esterification of Example 1. The result of the scent evaluation of the obtained propyl 1-ethylcyclohexanecarboxylate is shown in Table 1.

### Propyl 1-Ethylcyclohexanecarboxylate

As a result of the GC analysis, the purity of the main product was 98.7%. It was confirmed that the result of the GC-MS analysis of the main product was 198 (5, M⁺), 170 (15), 157 (18), 143 (12), 111 (100), 101 (20), 69 (68), 55 (23), and it matched a molecular weight of the target product of 198. Furthermore, the analysis value of ¹H-NMR (CDCl₃, 400 MHz, δ ppm) was 0.80 (t, J = 7.6 Hz, 3H), 0.95 (t, J = 7.4 Hz, 3H), 1.11-1.39 (m, 5H), 1.47-1.71 (m, 7H), 2.02-2.12 (m, 2H), 4.04 (t, J = 6.6 Hz, 2H), and the analysis value of ¹³C-NMR (CDCl₃, 400 MHz, δ ppm) was 8.5 (CH₃), 10.6 (CH₃), 22.1 (CH₂), 23.3 (CH₂), 26.1 (CH₂), 33.3 (CH₂), 33.8 (CH₂), 47.4 (C), 65.6 (OCH₂), 176.9 (CO), and the main product was identified as propyl 1-ethylcyclohexanecarboxylate.

### Example 4 (Production of Isopropyl 1-Ethylcyclohexanecarboxylate)

71 g of isopropyl 1-ethylcyclohexanecarboxylate as a colorless, transparent liquid was obtained in the same manner as in Example 1 except for using 46 g (0.76 mol) of 2-propanol in place of 41.7 g (0.9 mol) of the ethanol used in the esterification of Example 1. The result of the scent evaluation of the obtained isopropyl 1-ethylcyclohexanecarboxylate is shown in Table 1.

### Isopropyl 1-Ethylcyclohexanecarboxylate

As a result of the GC analysis, the purity of the main product was 99.9%. It was confirmed that the result of the GC-MS analysis of the main product was 198 (5, M⁺), 170 (15), 111 (100), 1013 (30), 69 (78), 55 (24), and it matched a molecular weight of the target product of 198. Furthermore, the analysis value of ¹H-NMR (CDCl₃, 400 MHz, δ ppm) was 0.80 (t, J = 7.6 Hz, 3H), 1.08-1.22 (m, 3H), 1.22 (d, J = 6.0 Hz, 6H), 1.26-1.40 (m, 2H), 1.46-1.61 (m, 5H), 2.02-2.10 (m, 2H), 5.04 (dq, 1H, J = 6.3 Hz, 6.3 Hz), and the analysis value of ¹³C-NMR (CDCl₃, 400 MHz, δ ppm) was 8.4 (CH₃), 21.9 (CH₃), 23.3 (CH₂), 23.3 (CH₂), 26.1 (CH₂), 33.4 (CH₂), 33.8 (CH₂), 47.0 (C), 66.9 (OCH₂), 176.2 (CO), and the main product was identified as isopropyl 1-ethylcyclohexanecarboxylate.

### [Table 1]

**Table 1**

| | Compound name | R¹ in Formula (1) | Scent evaluation |
|---|---|---|---|
| Example 1 | Ethyl 1-ethylcyclohexanecarboxylate | Ethyl group | Herbal scent with especially good diffusibility and fruity scent achieved |
| Example 2 | Methyl 1-ethylcyclohexanecarboxylate | Methyl group | Herbal scent with especially good diffusibility achieved |
| Example 3 | Propyl 1-ethylcyclohexanecarboxylate | n-Propyl group | Herbal scent with especially good diffusibility achieved |
| Example 4 | Isopropyl 1-ethylcyclohexanecarboxylate | Isopropyl group | Herbal scent with especially good diffusibility achieved |

It was found that the 1-ethylcyclohexanecarboxylic acid ester obtained in each of Examples 1 to 4 had an herbal scent and diffusibility of the scent and was able to be suitably used as a fragrance. It was further found that the 1-ethylcyclohexanecarboxylic acid ester obtained in each of Examples 1 to 4 was able to be suitably used for a perfuming method for imparting the herbal scent. In particular, it was found that the ethyl 1-ethylcyclohexanecarboxylate obtained in Example 1 was able to be suitably used for a fragrance because the ethyl 1-ethylcyclohexanecarboxylate also had a fruity scent in addition to the herbal scent.

### Examples 5 to 8 and Comparative Example 1 (Fragrance Composition)

A floral-type fragrance composition containing each of the 1-ethylcyclohexanecarboxylic acid esters obtained in Examples 1 to 4 as a blended component was prepared. Note that, as a comparative example, a fragrance composition in which the 1-ethylcyclohexanecarboxylic acid ester had been replaced with dipropylene glycol, which was a solvent, was prepared. The compositions of the floral-type fragrance compositions are listed in Table 2. The results of the scent evaluation of the floral-type fragrance compositions are listed in Table 3.

### [Table 2]

**Table 2**

| Blended component | (parts by mass) | | | | |
|---|---|---|---|---|---|
| | Comparative Example 1 | Example 5 | Example 6 | Example 7 | Example 8 |
| Ethyl 1-ethylcyclohexanecarboxylate (Example 1) | - | 50 | - | - | - |
| Methyl 1-ethylcyclohexanecarboxylate (Example 2) | - | - | 50 | - | - |
| Propyl 1-ethylcyclohexanecarboxylate (Example 3) | - | - | - | 50 | - |
| Isopropyl 1-ethylcyclohexanecarboxylate (Example 4) | - | - | - | - | 50 |
| Dipropylene glycol | 233 | 183 | 183 | 183 | 183 |
| Dihydromyrcenol | 50 | 50 | 50 | 50 | 50 |
| Triplal ¹⁾ | 4 | 4 | 4 | 4 | 4 |
| Magnol ²⁾ | 2 | 2 | 2 | 2 | 2 |
| Undecavertol ³⁾ | 2 | 2 | 2 | 2 | 2 |
| o-tert-Butylcyclohexyl acetate | 20 | 20 | 20 | 20 | 20 |
| Poirenate ⁴⁾ | 2 | 2 | 2 | 2 | 2 |
| Raspberry ketone | 5 | 5 | 5 | 5 | 5 |
| Fruitate ⁵⁾ | 2 | 2 | 2 | 2 | 2 |
| Tricyclodecenyl propionate | 100 | 100 | 100 | 100 | 100 |
| Geraniol | 100 | 100 | 100 | 100 | 100 |
| Rose oxide | 1 | 1 | 1 | 1 | 1 |
| α-Hexyl cinnamic aldehyde | 100 | 100 | 100 | 100 | 100 |
| Methyl dihydrojasmonate | 50 | 50 | 50 | 50 | 50 |
| Dimethyl anthranilate | 5 | 5 | 5 | 5 | 5 |
| Methyl β-naphthyl ketone | 5 | 5 | 5 | 5 | 5 |
| Lilial ⁶⁾ | 100 | 100 | 100 | 100 | 100 |
| Linalool | 50 | 50 | 50 | 50 | 50 |
| Terpineol | 50 | 50 | 50 | 50 | 50 |
| Phenylacetaldehyde dimethyl acetal | 4 | 4 | 4 | 4 | 4 |
| Amber core ⁷⁾ | 30 | 30 | 30 | 30 | 30 |
| Isobornyl cyclohexanol | 20 | 20 | 20 | 20 | 20 |
| Galaxolide 50% IPM ⁸⁾ | 60 | 60 | 60 | 60 | 60 |
| Ambroxan ⁹⁾ 5% DPG ¹⁰⁾ | 4 | 4 | 4 | 4 | 4 |
| Heliotropylacetone | 1 | 1 | 1 | 1 | 1 |
| Total | 1000 | 1000 | 1000 | 1000 | 1000 |

| | | | | | |
|---|---|---|---|---|---|
| 1) Triplal: IFF (product name); compound name: 2,4-dimethyl-3-cyclohexene-1-carboxyaldehyde 2) Magnol: Kao Corporation (product name), a mixture mainly containing ethyl norbornylcyclohexanol 3) Undecavertol: Givaudan (product name); compound name: 4-methyl-3-decen-5-ol 4) Poirenate: Kao Corporation (trade name); compound name: ethyl 2-cyclohexylpropionate 5) Fruitate: Kao Corporation (trade name); compound name: ethyl tricyclo[5.2.1.0^{2,6}]decane-2-carboxylate 6) Lilial: Givaudan (product name); compound name: p-tert-butyl-α-methylhydrocinnamic aldehyde 7) Amber core: Kao Corporation (trade name); compound name: 1-(2-tert-butylcyclohexyloxy)-2-butanol 8) 50% IPM: 50% isopropyl myristate (IPM) solution 9) Ambroxan: Kao Corporation (trade name); compound name: dodecahydro-3a,6,6,9a-tetramethylnaphtho[2,1-b]furan 10) 5% DPG: 5% dipropylene glycol (DPG) solution | | | | | |

### [Table 3]

**Table 3**

| | Added 1-ethylcyclohexanecarboxylic acid ester | R¹ in Formula (1) | Scent evaluation of floral-type fragrance composition |
|---|---|---|---|
| Comparative Example 1 | None (dipropylene glycol) | - | Floral-like |
| Example 5 | Ethyl 1-ethylcyclohexanecarboxylate (Example 1) | Ethyl group | Floral-like with improved diffusibility and natural feel, impressions of herbal feel and fruity feel imparted |
| Example 6 | Methyl 1-ethylcyclohexanecarboxylate (Example 2) | Methyl group | Floral-like with improved diffusibility, impression of herbal feel imparted |
| Example 7 | Propyl 1-ethylcyclohexanecarboxylate (Example 3) | n-Propyl group | Floral-like with improved diffusibility, impression of herbal feel imparted |
| Example 8 | Isopropyl 1-ethylcyclohexanecarboxylate (Example 4) | Isopropyl group | Floral-like with improved diffusibility, impression of herbal feel imparted |

### Example 9 and Comparative Example 2 (Fragrance Composition)

A fragrance composition having a sweet pea scent and containing the ethyl 1-ethylcyclohexanecarboxylate obtained in Example 1 as a blended component was prepared. Note that, as a comparative example, a fragrance composition in which the 1-ethylcyclohexanecarboxylic acid ester had been replaced with dipropylene glycol, which was a solvent, was prepared. The compositions of the fragrance compositions having sweet pea scents are listed in Table 4. The results of the scent evaluation of the fragrance compositions having sweet pea scents are listed in Table 5.

### [Table 4]

**Table 4**

| Blended component | | |
|---|---|---|
| | Comparative Example 2 | Example 9 |
| Ethyl 1-ethylcyclohexanecarboxylate (Example 1) | - | 12 |
| Dipropylene glycol | 203.8 | 191.8 |
| AMBERONE (ISO E SUPER (trade name)) | 30 | 30 |
| AMBROXAN (trade name) | 0.2 | 0.2 |
| BENZYL ACETATE | 5 | 5 |
| CIS-3-HEXENOL | 2 | 2 |
| CITRONELLOL | 50 | 50 |
| DAMASCONE BETA | 0.5 | 0.5 |
| ETHYL LINALOOL | 20 | 20 |
| EXALTOLIDE TOTAL | 20 | 20 |
| FLOROL (trade name) | 50 | 50 |
| GERANIOL | 30 | 30 |
| GERANYL ACETATE | 10 | 10 |
| HEDIONE (trade name) | 150 | 150 |
| HELIONAL (trade name) | 5 | 5 |
| HELIOTROPINE | 5 | 5 |
| HEXYL SALICILATE | 60 | 60 |
| INDOLAROME | 0.5 | 0.5 |
| IONONE BETA | 20 | 20 |
| LINALOOL (trade name) | 50 | 50 |
| LINALYL ACETATE | 15 | 15 |
| METHYL ISO EUGENOL | 5 | 5 |
| MUSK 50 IPM (GALAXOLIDE (trade name) 50 IPM) | 140 | 140 |
| PHENOXANOL | 50 | 50 |
| PHENYL ETHYL ALCOHOL | 20 | 20 |
| ROSE OXIDE | 1 | 1 |
| SANDALORE (trade name) | 2 | 2 |
| STYRALLYL ACETATE | 5 | 5 |
| TERPINEOL | 50 | 50 |
| Total | 1000 | 1000 |

### [Table 5]

**Table 5**

| | Added 1-ethylcyclohexanecarboxylic acid ester | R¹ in Formula (1) | Scent evaluation of fragrance composition having sweet pea scent |
|---|---|---|---|
| Comparative Example 2 | None (dipropylene glycol) | - | Scent of sweet pea without diffusibility |
| Example 9 | Ethyl 1-ethylcyclohexanecarboxylate (Example 1) | Ethyl group | Scent of sweet pea with sharp top note and diffusibility |

### Example 10 and Comparative Example 3 (Fragrance Composition)

A peach-type fragrance composition containing the ethyl 1-ethylcyclohexanecarboxylate obtained in Example 1 as a blended component was prepared. Note that, as a comparative example, a fragrance composition in which the 1-ethylcyclohexanecarboxylic acid ester had been replaced with dipropylene glycol, which was a solvent, was prepared. The compositions of the peach-type fragrance compositions are listed in Table 6. The results of the scent evaluation of the peach-type fragrance compositions are listed in Table 7.

### [Table 6]

**Table 6**

| Blended component | | |
|---|---|---|
| | Comparative Example 3 | Example 10 |
| Ethyl 1-ethylcyclohexanecarboxylate (Example 1) | - | 40 |
| Dipropylene glycol | 709 | 669 |
| ALDEHYDE C-14 | 4 | 4 |
| ALLYL IONONE | 5 | 5 |
| BENZYL ACETATE | 30 | 30 |
| CIS-3-HEXENOL | 0.5 | 0.5 |
| D.M.P.E CARBINOL | 100 | 100 |
| DAMASCONE BETA | 2 | 2 |
| DECALACTONE GAMMA | 5 | 5 |
| ETHYL LINALOOL | 40 | 40 |
| GERANIOL | 20 | 20 |
| IONONE BETA | 20 | 20 |
| LIGUSTRAL | 4 | 4 |
| LINALOOL OXIDE | 20 | 20 |
| METHYL ISO EUGENOL | 40 | 40 |
| VANILLIN | 0.5 | 0.5 |
| Total | 1000 | 1000 |

### [Table 7]

**Table 7**

| | Added 1-ethylcyclohexanecarboxylic acid ester | R¹ in Formula (1) | Scent evaluation of peach-type fragrance composition |
|---|---|---|---|
| Comparative Example 3 | None (dipropylene glycol) | - | Scent of peach with little impression of fruity feel, without diffusibility |
| Example 10 | Ethyl 1-ethylcyclohexanecarboxylate (Example 1) | Ethyl group | Scent of peach with light, fruity sweetness and diffusibility |

The fragrance compositions of Examples each had an impression of an herbal feel and had improved diffusibility of the scent compared to the fragrance composition of Comparative Example. As in Tables 3, 5, and 7, because the fragrance compositions of Examples had excellent aroma characteristics and diffusibility, the fragrance compositions of Examples are especially useful as perfuming components for a wide variety of toiletry products, such as detergents for clothing and detergents for bodies. In particular, the fragrance composition containing the ethyl 1-ethylcyclohexanecarboxylate as an active component is remarkably useful as a perfuming component because the fragrance composition has an impression of a fruity feel with excellent diffusibility, as shown in Examples 5 and 10.

Based on the results described above, it was found that the fragrance compositions of Examples each had an impression of an herbal feel and excellent diffusibility of the scent. Furthermore, it was found that the specific 1-ethylcyclohexanecarboxylic acid ester used in Examples has an herbal scent and, in addition, can impart diffusibility of the scent, and thus can be used as a fragrance. In addition, it was found that the specific 1-ethylcyclohexanecarboxylic acid ester used in Examples can be suitably used for a perfuming method for imparting an herbal scent.

## Claims

1. A fragrance composition, comprising a 1-ethylcyclohexanecarboxylic acid ester represented by General Formula (1) as an active component: 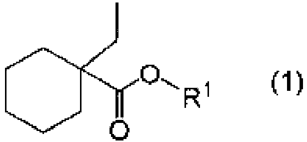 where in Formula (1), R¹ represents an alkyl group having from 1 to 3 carbons.

2. The fragrance composition according to claim 1, further comprising a fragrance substance other than the 1-ethylcyclohexanecarboxylic acid ester represented by Formula (1).

3. The fragrance composition according to claim 2, wherein the fragrance substance other than the 1-ethylcyclohexanecarboxylic acid ester represented by Formula (1) is at least one selected from the group consisting of hydrocarbons, alcohols, phenols, aldehydes, ketones, acetals, ketals, ethers, nitriles, lactones, natural essential oils, natural extracts, and esters other than the 1-ethylcyclohexanecarboxylic acid ester represented by Formula (1).

4. The fragrance composition according to any one of claims 1 to 3, wherein R¹ is at least one selected from the group consisting of a methyl group, an ethyl group, an n-propyl group, and an isopropyl group.

5. The fragrance composition according to any one of claims 1 to 4, wherein R¹ is an ethyl group.

6. Use of a 1-ethylcyclohexanecarboxylic acid ester represented by General Formula (1) as a fragrance: 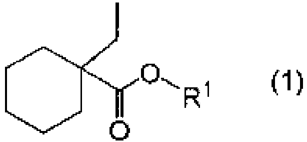 where in Formula (1), R¹ represents an alkyl group having from 1 to 3 carbons.

7. The use according to claim 6, wherein R¹ is at least one selected from the group consisting of a methyl group, an ethyl group, an n-propyl group, and an isopropyl group.

8. The use according to claim 6 or 7, wherein the 1-ethylcyclohexanecarboxylic acid ester represented by Formula (1) imparts an herbal scent.

9. The use according to any one of claims 6 to 8, wherein R¹ is an ethyl group.

10. The use according to claim 9, wherein the 1-ethylcyclohexanecarboxylic acid ester represented by Formula (1) imparts an herbal scent and a fruity scent.

11. A perfuming method for imparting an herbal scent by a 1-ethylcyclohexanecarboxylic acid ester represented by Formula (1a): 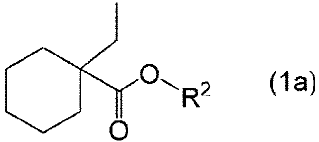 where in Formula (1a), R² represents at least one selected from the group consisting of a methyl group, an ethyl group, an n-propyl group, and an isopropyl group.

12. The perfuming method according to claim 11, wherein R² is an ethyl group, and the 1-ethylcyclohexanecarboxylic acid ester represented by Formula (1a) imparts an herbal scent and a fruity scent.

13. A method for producing a 1-ethylcyclohexanecarboxylic acid ester, the method comprising reacting a compound represented by General Formula (2) with carbon monoxide and an alcohol represented by General Formula (3) in the presence of hydrogen fluoride to obtain a 1-ethylcyclohexanecarboxylic acid ester represented by General Formula (1): 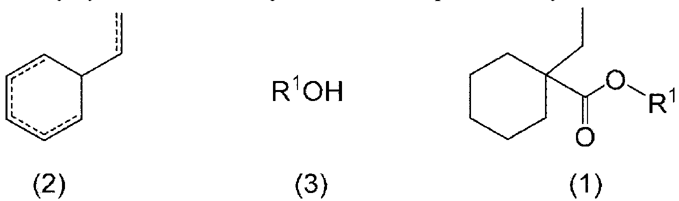 where in Formula (2), among carbon-carbon bonds with broken lines, one is a double bond, and the other bonds are single bonds; in Formula (3), R¹ represents an alkyl group having from 1 to 3 carbons; and in Formula (1), R¹ is the same as R¹ in Formula (3) and represents an alkyl group having from 1 to 3 carbons.

14. The method for producing a 1-ethylcyclohexanecarboxylic acid ester according to claim 13, wherein R¹ is at least one selected from the group consisting of a methyl group, an ethyl group, an n-propyl group, and an isopropyl group.

15. The method for producing a 1-ethylcyclohexanecarboxylic acid ester according to claim 13 or 14, wherein R¹ is an ethyl group.

16. The method for producing a 1-ethylcyclohexanecarboxylic acid ester according to any one of claims 13 to 15, wherein the compound represented by General Formula (2) is at least one selected from the group consisting of vinylcyclohexane and 4-ethylcyclohexene.

17. The method for producing a 1-ethylcyclohexanecarboxylic acid ester according to any one of claims 13 to 16, wherein the method comprises obtaining 4-ethylcyclohexene by subjecting 4-vinylcyclohexene to hydrogenation, and the compound represented by General Formula (2) is 4-ethylcyclohexene.
